# DEMANDE DE BREVET EUROPEEN

(11) **EP 2 682 151 A1**
(43) Date de publication de la demande: **08.01.2014**
(21) Numéro de dépôt: 13172887.5
(22) Date de dépôt: 19.06.2013
(51) Int. Cl.: A61M 25/00, A61N 1/05, B29C 47/00, A61M 25/01

(54) **Microcathéter implantable dans des réseaux veineux, artériels ou lymphatiques**

(30) Priorité: 06.07.2012 FR 1256516
(71) Demandeur: Sorin CRM SAS, 92140 Clamart (FR)
(72) Inventeur: Régnier, Willy, 91160 Longjumeau (FR); Ollivier, Jean-François, 91190 Villiers le Bacle (FR); Shan, Nicolas, 91260 Juvisy sur Orge (FR)
(74) Mandataire: Dupuis-Latour, Dominique

(57) **Abrégé**

Ce microcathéter comprend un tube creux (14) avec une lumière centrale s'étendant sur toute la longueur du tube depuis une région proximale (26) jusqu'à une région distale (22). Le diamètre externe du tube du cathéter dans la région distale (22) est au plus égal à 1 mm (3 French), le diamètre de la lumière centrale du tube du cathéter étant au moins égal à 0,3 mm. La raideur en flexion de la région proximale (26) est supérieure à la raideur en flexion de la région distale (22), et le tube comporte une région de transition (24) présentant un gradient de raideur décroissant depuis la région proximale (26) vers la région distale (22). La paroi du tube est dépourvue d'armure au moins dans la région distale, et le cathéter est réalisé en matériau(x) biocompatible(s) adapté(s) à une implantation permanente dans des réseaux veineux, artériels ou lymphatiques, et porte dans la région distale des éléments radio-opaques.

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation et/ou de resynchronisation en cas de trouble du rythme détecté par l'appareil. Elle inclut aussi les appareils neurologiques, les implants cochléaires, les pompes de diffusion de substances médicales, les capteurs biologiques implantés, etc.

Ces dispositifs comportent un boitier généralement désigné "générateur", raccordé électriquement et mécaniquement à une ou plusieurs "sondes" intracorporelles munie(s) d'électrodes destinées à venir en contact avec les tissus sur lesquels on souhaite appliquer des impulsions de stimulation et/ou recueillir un signal électrique : myocarde, nerf, muscle, ...

La sonde est un objet implanté au travers de divers vaisseaux, notamment veineux, artériels ou lymphatiques, et sa fonction est de transmettre un signal électrique au tissu-cible et/ou d'y recueillir un potentiel électrique, typiquement un potentiel résultant d'une onde de dépolarisation cardiaque.

La tendance clinique dans le domaine des sondes implantables est d'en réduire la taille afin de les rendre moins invasives et plus faciles à manipuler à travers les vaisseaux, la taille des sondes implantables étant aujourd'hui typiquement de l'ordre de 4 à 6 French (1,33 à 2 mm).

Au-delà de ces limites, on se heurte toutefois à une très grande augmentation de la complexité de conception et de fabrication du fait de multiples contraintes techniques à prendre en compte, de surcroit génératrices de risques nouveaux.

Pourtant, une telle réduction, à moins de 2 French (0,66 mm) par exemple, ouvrirait des perspectives d'applications médicales dans des domaines variés, allant de la cardiologie à la neurologie, en présence d'un réseau veineux, artériel ou même lymphatique comme le réseau veineux cérébral ou le réseau veineux du sinus coronaire.

En neurologie notamment, les techniques de stimulation électrique permettent aujourd'hui des avancées importantes dans le domaine de la neuromodulation, technique qui consiste à stimuler des zones-cibles du cerveau pour le traitement de la maladie de Parkinson, l'épilepsie et autres maladies neurologiques. On pourrait donc envisager de traiter de cette manière de nouvelles régions-cibles difficilement atteignables aujourd'hui à travers le réseau de vaisseaux, ceci au moyen de sondes de stimulation de petite taille, ci-après désignées "microsondes", présentant une grande robustesse afin de garantir la biostabilité à long terme. Une telle technique permettrait une approche moins invasive de ces traitements et surtout une efficacité supérieure des traitements administrés.

La mise en place de ces "microsondes" et leur positionnement précis au plus profond de la zone cible (réseau coronaire profond, réseau cérébral, ...) posent cependant des problèmes restés jusqu'à présent sans solution, en particulier du fait de leur très grande souplesse.

On connait certes les "fils-guides", qui sont des accessoires à structure effilée qui présentent une raideur importante dans la partie proximale et faible dans leur partie distale. Ils peuvent atteindre des cibles lointaines dans les réseaux biologiques artériels, veineux ou même lymphatiques, parfois en franchissant des valves et tortuosités particulièrement étroites. En revanche, leur structure ne présente pas une résistance en fatigue suffisamment élevée pour être compatible avec les exigences particulièrement strictes des implants permanents. En effet, à la différence d'un fil-guide, une microsonde est très souple et difficile à mettre en place :
- sa raideur en torsion est insuffisante pour permettre la transmission sur toute la longueur du corps de sonde, jusqu'à l'extrémité distale, d'un mouvement de rotation imprimé par la poignée de manoeuvre depuis l'extrémité proximale (manque de "torquabilité") ; et
- il est impossible de la faire progresser dans le réseau biologique sans arc-boutement, en la poussant depuis l'extrémité proximale au moyen de la poignée de manoeuvre (manque de "pushabilité").

La conception d'une microsonde de très faible diamètre (typiquement, de diamètre inférieur à 2F, soit 0,66 mm) implique donc de résoudre plusieurs difficultés :
- permettre la mise en place d'une microsonde qui, en elle-même, ne présente que de faibles performances en navigabilité à travers les réseaux biologiques, du fait du manque de "pushabilité" et de "torquabilité" en particulier ;
- autoriser la mise en place de cette microsonde malgré la présence à l'extrémité proximale d'un connecteur de grand diamètre tel qu'un connecteur normalisé IS-1 ou IS-4 (présentant typiquement un diamètre supérieur à 10 F, soit 3,3 mm), car ce plus grand diamètre empêcherait de retirer un cathéter de rigidification dans lequel la microsonde flexible aurait été préalablement enfilée ;
- protéger la microsonde des risques de compression et des risques de blessure du corps de sonde, notamment au niveau de la zone de manipulation par le chirurgien et de la connexion avec le boitier, et la protéger également contre les risques d'abrasion au contact d'autres dispositifs ;
- éventuellement, aider à la stabilisation de la microsonde en supportant celle-ci au niveau des accès par le réseau veineux, jusqu'à l'entrée dans le sinus coronaire pour le cas d'une microsonde implantée dans le réseau coronaire veineux ; et
- permettre, par injection de produit de contraste dans la lumière interne du microcathéter ou dans une lumière déportée, de visualiser les microveines par l'intermédiaire d'une technique de visualisation aux rayons X, très usuelle lors de l'implantation de microsondes dans des veines ne mesurant que 1 ou 2 F (0,33 ou 0,66 mm).

Le but de l'invention est de résoudre ces problèmes en proposant une solution de mise en place d'une microsonde qui procure à la sonde : i) une excellente navigabilité à travers les réseaux biologiques, ii) une raideur suffisamment faible dans la région distale pour pouvoir suivre les vaisseaux les plus tortueux, et iii) des propriétés de "pushabilité" et de "torquabilité" permettant de transmettre les efforts de poussée axiale et de torsion imprimés par le chirurgien depuis l'extrémité proximale lors de la mise en place.

Le US 2004/153049 A1 décrit un microcathéter dont l'extrémité distale est suffisamment fine pour pénétrer dans des vaisseaux profonds de très petit calibre. Ce document divulgue une structure tubulaire renforcée par une armure tressée prise en sandwich entre deux couches polymère, le tout étant conformé et dimensionné de manière à présenter un gradient de raideur décroissante en direction de l'extrémité distale.

Le WO 2010/068793 A1 décrit un microcathéter destiné à des manoeuvres endovasculaires, comprenant une armure bobinée protégée côté intérieur et côté extérieur par un chemisage en matériau polymère. Le cathéter est constitué de plusieurs segments plus ou moins flexibles assemblés en succession, de manière à procurer à l'ensemble un gradient de raideur entre l'extrémité proximale et l'extrémité distale.

Les EP 2 062 609 A1, US 5 308 342 A et WO 98/50097 A1 divulguent encore d'autres types de cathéters à raideur variable, comprenant diverses structures de renforcement à l'encontre des sollicitations mécaniques rencontrées lors de leur utilisation.

L'objet de l'invention est une structure de cathéter qui soit spécifiquement adaptée à l'opération d'implantation d'une microsonde, cathéter qui sera par la suite désigné "microcathéter".

Un premier but de l'invention est de proposer un tel microcathéter qui réponde aux contraintes générales énumérées plus haut, pour pouvoir notamment atteindre des veinules de très petit diamètre, inaccessibles aujourd'hui avec des dispositifs de taille supérieure. Le microcathéter de l'invention devra également faciliter sensiblement la navigabilité dans les réseaux veineux artériels ou lymphatiques avec des caractéristiques de souplesse adaptées, amplifiées par un diamètre réduit.

L'utilisation d'un tel microcathéter pour implanter une microsonde introduit toutefois une difficulté supplémentaire.

En effet, comme on l'a indiqué plus haut, la microsonde est pourvue à son extrémité proximale d'une fiche de connecteur, qui empêche le retrait du cathéter une fois la microsonde mise en place.

Avec une sonde conventionnelle, pour contourner cette difficulté on utilise un cathéter découpable ou pelable, scindé en deux parties par incision ou arrachement le long d'une génératrice du tube de cathéter.

Mais l'extrême finesse d'un microcathéter ne permet pas de mettre en oeuvre cette technique.

De ce fait, et selon un aspect original de l'invention, il est proposé de laisser en place le microcathéter après implantation de la microsonde, en conférant à celui-ci des propriétés d'implantabilité que ne possèdent pas les cathéters conventionnels utilisés pour ces interventions (cathéters qui ne servent que le temps de la pose).

Par "implantabilité", on entendra l'aptitude à une implantation permanente dans le réseau veineux, artériel ou même lymphatique, en toute sécurité pour le patient tant au niveau du matériau choisi, qui doit être biocompatible et notamment hémocompatible, que de l'absence de risques mécaniques, et ceci pendant une durée pouvant atteindre plusieurs années. Un microcathéter présentant ces propriétés sera par la suite dénommé "microcathéter implantable".

Ce caractère "implantable" est entendu notamment au sens de la directive précitée 90/385/CEE du Conseil des communautés européennes du 20 juin 1990, selon laquelle est qualifié d"'implantable" tout dispositif médical actif *"conçu pour être implanté en totalité ou en partie, par une intervention chirurgicale ou médicale dans le corps humain ou, par une intervention médicale, dans un orifice naturel, et qui est destiné à rester après l'intervention".* La directive 93/42/CEE du Conseil des communautés européennes du 14 juin 1993 relative aux dispositifs médicaux en général donne une définition comparable, avec une généralisation à tout dispositif même partiellement introduit dès lors qu'il est *"destiné à demeurer en place après l'intervention pendant une période d'au moins trente jours".*

En particulier, pour répondre à cette contrainte d'implantabilité, le microcathéter devra pouvoir résister sans rupture typiquement à 400 000 000 de sollicitations en flexion, valeur correspondant au nombre moyen de battements cardiaques sur la durée de vie nominale de la microsonde (10 ans).

La durée de vie est en effet un paramètre fondamental qui doit être absolument pris en compte lors de la conception de tout dispositif implantable, tel que le microcathéter objet de l'invention. En effet, les battements du coeur et les mouvements des organes induisent sur ce type de dispositif des déformations de flexion qui doivent être parfaitement maitrisées, sans risque de fatigue du matériau pouvant entrainer sa rupture et limiter ainsi sa durée de vie.

Au surplus, une microsonde et un microcathéter introduits par exemple dans le réseau veineux profond peuvent être le siège de déformations suivant une courbure beaucoup plus forte que pour une sonde ou un cathéter normal, dans la mesure où ces dispositifs doivent suivre les déformations et tortuosités des veines profondes. Cette courbure plus importante engendre dans le matériau, toutes choses égales par ailleurs, des contraintes plus importantes qui rendent la résistance à la fatigue encore plus critique.

Au surplus, dans l'hypothèse où un tel microcathéter viendrait quand même à se rompre, la structure du microcathéter ne doit créer dans une telle éventualité aucun risque pour le patient : ceci exclut les structures de cathéter conventionnelles telles que celles divulguées par les documents cités plus haut, qui intègrent une armure de rigidification, potentiellement dangereuse en cas de rupture.

En résumé et en d'autres termes, il s'agit de pouvoir disposer d'un microcathéter capable de passer les tests d'implantabilité permanente jusqu'à présent réservés aux sondes, en particulier :
- biocompatibilité garantie selon ISO 10993 (dispositifs médicaux en général) et EN 45502 (concernant plus particulièrement les sondes) ;
- absence de corrosion ;
- absence de risque de rupture ;
- en cas de rupture, innocuité pour le patient.

L'invention propose un microcathéter du type général divulgué par le US 2004/153049 A1 précité, c'est-à-dire un dispositif comprenant un tube creux avec une lumière centrale s'étendant sur toute la longueur du tube depuis une région proximale jusqu'à une région distale. Le diamètre externe du tube du cathéter dans la région distale est au plus égal à 1 mm (3 French) et le diamètre de la lumière centrale du tube du cathéter est au moins égal à 0,2 mm. La raideur en flexion de la région proximale est supérieure à la raideur en flexion de la région distale, et le tube comporte une région de transition présentant un gradient de raideur décroissant depuis la région proximale vers la région distale. Le cathéter porte dans la région distale des éléments radio-opaques ou des références visuelles, De façon caractéristique de l'invention, ce cathéter présente des propriétés lui permettant d'être considéré comme un "microcathéter implantable" au sens indiqué plus haut, qui sont les suivantes : la paroi du tube est dépourvue d'armure de rigidification ; la région distale présente une raideur constante ; et le cathéter est réalisé en matériau(x) biocompatible(s) adapté(s) à une implantation permanente dans des réseaux veineux, artériels ou lymphatiques.

Selon diverses caractéristiques subsidiaires avantageuses :
- le tube présente un gradient d'épaisseur décroissante, de la région proximale vers la région distale du cathéter ;
- le tube comporte une portion de diamètre extérieur décroissant de la région proximale vers la région distale du cathéter, le diamètre intérieur de la lumière centrale étant constant sur toute la longueur du tube ;
- le tube comprend un noyau tubulaire de diamètre extérieur constant revêtu d'un matériau de gainage d'épaisseur variable, cette épaisseur augmentant progressivement de la région distale vers la région proximale du cathéter ;
- la raideur en flexion de la partie distale n'est pas supérieure à 0,09 N/mm ;
- le ratio entre la raideur en flexion de la partie proximale et la raideur en flexion de la partie distale est compris entre 2 et 10 ;
- le tube présente dans la direction de sa longueur une structure sans discontinuité et sans soudure ou bien, en variante, il comprend dans la direction de sa longueur une structure formée d'une série de tubes de diamètres respectifs différents, réunis les uns à la suite des autres ;
- la portion de diamètre extérieur décroissant est une portion conique de diamètre uniformément décroissant ;
- la portion de diamètre extérieur décroissant est une portion médiane du cathéter, prolongée côté proximal par une portion de diamètre constant et côté distal par une portion de diamètre constant, l'épaisseur et le diamètre extérieur du tube dans la région proximale étant supérieurs à l'épaisseur et au diamètre extérieur du tube dans la région distale ;
- le cathéter comprend en outre dans la région proximale un organe de solidarisation à un corps d'une sonde apte à être introduite dans la lumière du cathéter, notamment un embout radialement compressible apte à recevoir une ligature, ou bien une pince comportant une pièce centrale de pincement pourvue de mors déformables, entourée par une pièce de serrage desdits mors ;
- la surface intérieure de la lumière centrale est une surface d'un matériau antifriction différent du matériau formant le reste du tube ;
- le cathéter porte en outre dans une région centrale du tube un manchon de retenue apte à assujettir le cathéter à la paroi d'un vaisseau dans une région distante de la région distale. ◇
On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre de façon générale le myocarde, avec les principales veines du réseau coronaire, où a été introduite une microsonde de stimulation d'une cavité gauche du coeur, associée à un microcathéter implantable selon l'invention.
La Figure 2 est une vue en coupe d'un autre exemple d'implantation du microcathéter selon l'invention, dans le réseau vasculaire cérébral.
La Figure 3 est une vue d'ensemble du microcathéter implantable de l'invention, en montrant les différentes parties constitutives.
La Figure 4 illustre, isolément, le tube à section variable à partir duquel est réalisé le microcathéter implantable de la Figure 3.
La Figure 5 est une coupe du tube de la Figure 4, fortement agrandi dans la direction radiale de manière à en montrer la structure interne, avec un noyau tubulaire et un revêtement d'épaisseur variable.
La Figure 6 est un graphique montrant les variations de raideur du tube des Figures 4 et 5 en fonction de la position sur ce tube, ainsi qu'à titre comparatif la caractéristique de raideur d'un mandrin (fil-guide) conventionnel.
La Figure 7 illustre une microsonde utilisable avec le microcathéter de l'invention.
La Figure 8 illustre la microsonde de la Figure 7, insérée dans le microcathéter de la Figure 3.
Les Figures 9a et 9b sont des vues de détail, respectivement en perspective et en coupe, du moyen de verrouillage par ligature de la microsonde sur le microcathéter.
Les Figures 10a et 10b illustrent une première variante, à vis, du moyen de verrouillage des Figures 9a et 9b.
Les Figures 11a et 11b illustrent une deuxième variante, à encliquetage, du moyen de verrouillage des Figures 9a et 9b.
La Figure 12 illustre, partiellement en coupe et en perspective, le détail de l'extrémité proximale, formant connecteur, du microcathéter.
La Figure 13 illustre un accessoire d'injection emboité sur l'extrémité proximale formant connecteur de la Figure 12.

On va maintenant décrire des exemples de réalisation du microcathéter selon l'invention.

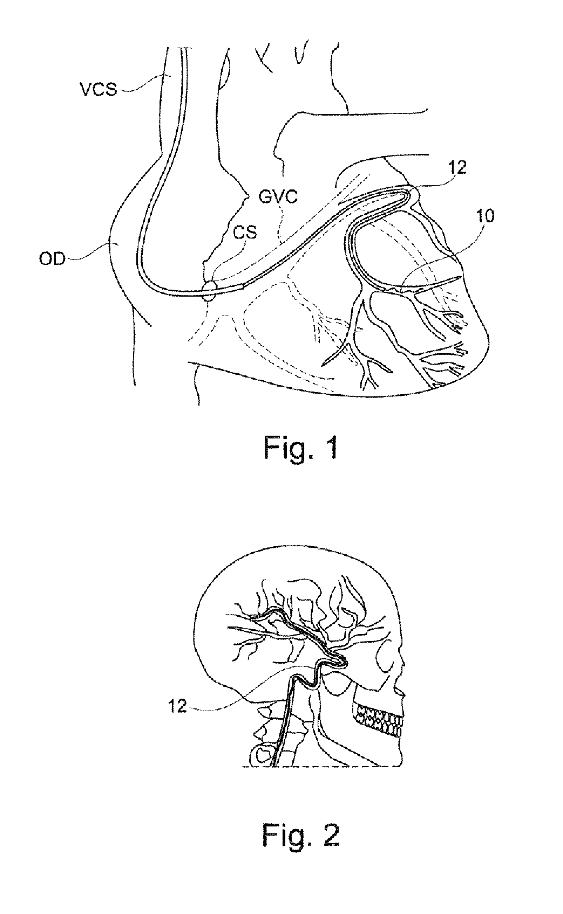

La Figure 1 illustre de façon générale le myocarde et les principaux vaisseaux du réseau coronaire dans lequel on a introduit une microsonde 10 au moyen d'un microcathéter implantable 12 selon l'invention, par voie endocavitaire via la veine cave supérieure VCS, l'oreillette droite OD, l'entrée CS du sinus coronaire veineux et la grande veine coronaire GVC.

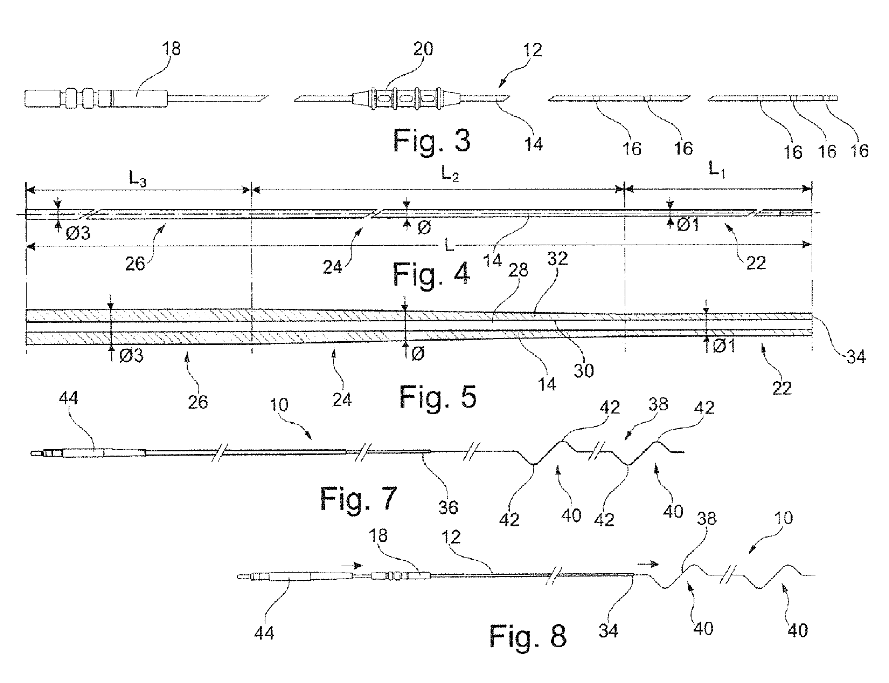

La microsonde 10 (qui sera décrite plus bas en détail en référence à la Figure 7) présente un diamètre au plus égal à 2 French (0,66 mm), typiquement de l'ordre de 0,5 à 2 French (0,16 à 0,66 mm). Elle porte une pluralité d'électrodes de détection/stimulation destinées notamment à stimuler le ventricule gauche à partir de plusieurs sites situés dans la veine coronaire.

Une telle microsonde est par exemple décrite dans le EP 2 455 131 A1 (Sorin CRM), auquel on pourra se référer pour de plus amples détails.

La microsonde 10 a été introduite dans le microcathéter 12 (cette configuration sera décrite plus bas en détail en référence à la Figure 8), qui pénètre dans le sinus coronaire CS et la grande veine cardiaque GVC puis dans le réseau coronaire profond jusqu'au voisinage de l'extrémité distale active de la microsonde 10, qui émerge de l'extrémité distale du microcathéter 12.

Si besoin, l'ensemble microsonde/microcathéter peut atteindre et traverser une anastomose. Une anastomose est un passage existant, à l'extrémité de certaines veines du réseau coronaire, vers une autre veine, avec de ce fait possibilité de communication entre deux veines distinctes au niveau de l'anastomose, via leurs extrémités distales respectives. Dans ce cas, l'ensemble microsonde/microcathéter est canulé dans une première veine (veine "aller") puis par l'anastomose vers une deuxième veine (veine "retour") en remontant dans celle-ci, ce qui permet de placer l'extrémité active de la microsonde dans des veines de très faible diamètre, non exploitées à ce jour du fait de la taille excessive des sondes coronaires permanentes conventionnelles.

Grâce à cette configuration, il est possible non seulement de stimuler le ventricule gauche à partir de points situés profondément dans une veine du réseau coronaire, mais également, via l'anastomose, dans des régions proximales de veines dans lesquelles il aurait été difficile de stabiliser ou fixer des sondes conventionnelles de stimulation du ventricule gauche, du fait du grand diamètre de l'embouchure de ces veines.

Le microcathéter 12 est, de façon caractéristique, apte :
- à pénétrer dans le réseau profond, donc présente une partie distale de diamètre très réduit ; et
- à être laissé en place après la pose de la microsonde (pour les raisons exposées plus haut, du fait de l'impossibilité de retirer un microcathéter), donc devant répondre à toutes les contraintes imposées à un accessoire présent à demeure dans le réseau veineux.

La Figure 3 illustre l'ensemble des éléments constituant le microcathéter 12.

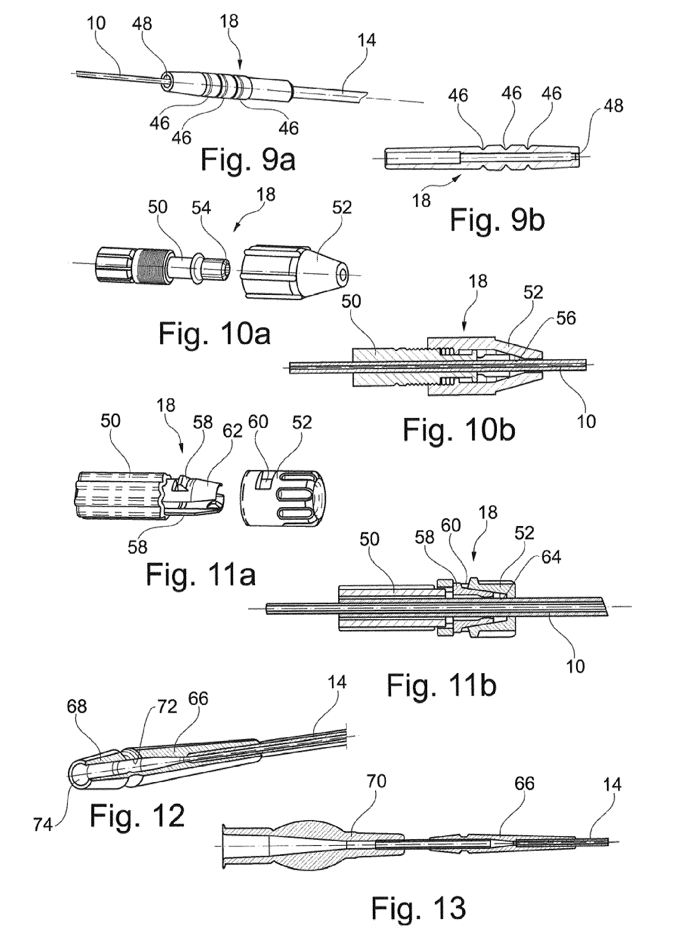

Ce microcathéter est constitué d'un tube polymère à section variable 14 (décrit plus en détail en référence aux Figures 4 et 5), portant à l'extrémité distale (à droite sur la figure) une série de bagues 16 radio-opaques, par exemple des bagues de platine ou de tout autre matériau noble biocompatible implantable (or, tantale, ...) permettant de visualiser la progression du microcathéter au moment de l'implantation. À l'extrémité proximale (à gauche sur la figure), le microcathéter 12 porte un élément 18, décrit plus en détail en référence aux Figures 9a et 9b, comportant notamment un système de serrage, par exemple par ligature, permettant en fin d'intervention de solidariser axialement au microcathéter la microsonde qui aura été introduite à l'intérieur de la lumière de ce microcathéter et d'éviter tout déplacement ultérieur de la microsonde.

Le microcathéter 12 comporte également, dans une région médiane, un manchon de fixation 20 permettant de l'immobiliser dans le réseau veineux du patient, au niveau sous-clavier.

On va maintenant décrire plus précisément, en référence aux Figures 4 et 5, la structure du tube 14 à partir duquel est réalisé le microcathéter 12 de l'invention.

Ce tube comprend une partie distale 22 de diamètre Ø₁ constant, par exemple Ø₁ = 0,8 mm (typiquement Ø₁ = 0,5 à 1,0 mm) et de longueur L₁ = 260 mm minimum (cette valeur dimensionnelle, tout comme celles qui seront indiquées par la suite, n'étant bien entendu pas limitative). Il comprend ensuite une partie médiane 14 de diamètre variable 0 et de longueur L₂ = 210 mm (ou moins) dans l'exemple illustré. Le microcathéter 12 comprend enfin une partie proximale 26 de diamètre Ø₃ constant, par exemple Ø₃ = 1,3 mm, et de longueur L₃ = 400 mm minimum dans cet exemple. La longueur totale L du tube est ainsi dans cet exemple de L = 870 mm.

La structure du cathéter 10 ne présente en direction longitudinale aucune discontinuité de type soudure ou collage, de manière à minimiser les risques de rupture en fatigue.

Le tube 14 est avantageusement réalisé par une technique d'extrusion à débit variable de deux matières avec, à l'intérieur, un tube 30 définissant la lumière interne 28, par exemple un tube en PTFE, matériau choisi pour ses propriétés de très faible coefficient de frottement (facilitant donc l'introduction et le déplacement de la microsonde 10 dans la lumière 28), sa capacité à être extrudé avec du polyuréthanne dans de très faibles épaisseurs, et sa souplesse mécanique.

À l'extérieur, le tube 30 en PTFE reçoit par extrusion un revêtement 32 par exemple de polyuréthanne, matériau choisi pour ses propriétés de souplesse, de résistance mécanique, de résistance à l'abrasion, de capacité à être extrudé et de biocompatibilité.

Le matériau peut être par exemple un polyuréthanne utilisable pour des applications d'implantation à long terme, ou bien un copolymère de polyuréthannes, un copolymère de polyuréthanne et de silicium, un polyéther bloc amide (par exemple de type *Pebax*), ou encore un polyamide. Ce matériau peut être chargé d'un opacifiant aux rayons X (BASO₄ ou TiO₂ par exemple) permettant de visualiser plus aisément le microcathéter sous amplificateur de brillance au moment de l'intervention. Il est possible de prévoir à la surface externe du microcathéter un revêtement biocompatible améliorant le glissement contre les parois des vaisseaux, par exemple un revêtement hydrophile de type polyvinylpyrrolidone (PVP) ou silicone. Le revêtement de surface peut également être choisi pour renforcer la biocompatibilité avec le milieu vivant, par ajout d'agents antimicrobiens hémocompatibles évitant la formation de thromboses, par exemple un revêtement d'un film mince de carbone.

Dans la partie médiane 24, le diamètre varie progressivement de Ø₁ = 0,8 mm à Ø₃ = 1,3 mm, et ce gradient de diamètre est obtenu par augmentation progressive de l'épaisseur du revêtement 32 (le diamètre du tube 30 étant constant) sous l'effet de la variation du débit d'extrusion. La loi de variation est une loi linéaire, ce qui donne à la partie médiane 24, du point de vue géométrique, une forme conique. L'extrémité distale 34 est bien entendu arrondie de façon appropriée afin de rendre le microcathéter atraumatique. Cette structure permet de minimiser les transitions de raideur le long du microcathéter, réduisant ainsi les concentrations de contraintes pouvant conduire à des fragilisations en fatigue du microcathéter. Dans une variante, le cathéter est formé d'une succession de tubes de différents diamètres, réunis les uns à la suite des autres (par exemple par collage, soudure thermique ou aux ultrasons, etc.) afin de réaliser le même assemblage.

La configuration que l'ont vient de décrire procure une très grande souplesse en partie distale (du fait de l'épaisseur réduite de la paroi du revêtement 32) et une plus grande rigidité en partie proximale (du fait de l'épaisseur plus importante de cette même paroi), et la transition d'une partie à l'autre est obtenue de façon continue, sans risque de création locale de contraintes excessives lors de la manipulation.

En particulier, la partie distale 22 du microcathéter est conçue de manière à présenter une raideur à la fois faible et constante, de manière à assurer l'excellente résistance à la fatigue nécessaire pour répondre aux critères d'implantabilité permanente (typiquement une résistance à 400 000 000 de sollicitations en flexion sans rupture, comme indiqué plus haut), en évitant toute concentration excessive de contraintes susceptibles de détériorer le microcathéter dans cette région où il est le plus fragile, du fait de son extrême finesse et de l'absence de toute armure de renfort (pour les raisons exposées plus haut, également liées à l'implantabilité permanente).

La caractéristique de raideur variable entre les parties distale et proximale 26 est rendue possible par des moyens de dégressivité de raideur ménagés entre la partie proximale 26 et la partie distale 22 du microcathéter, dans une partie médiane 24 formant une région de transition. Il est possible de définir le gradient de raideur progressif le long de la microsonde de manière à disposer (i) d'une part, d'une partie distale 22 souple, non traumatique, pouvant passer au travers des tortuosités et anastomoses, et (ii), d'autre part, d'une partie proximale 26 plus rigide permettant de transmettre la poussée exercée par le chirurgien.

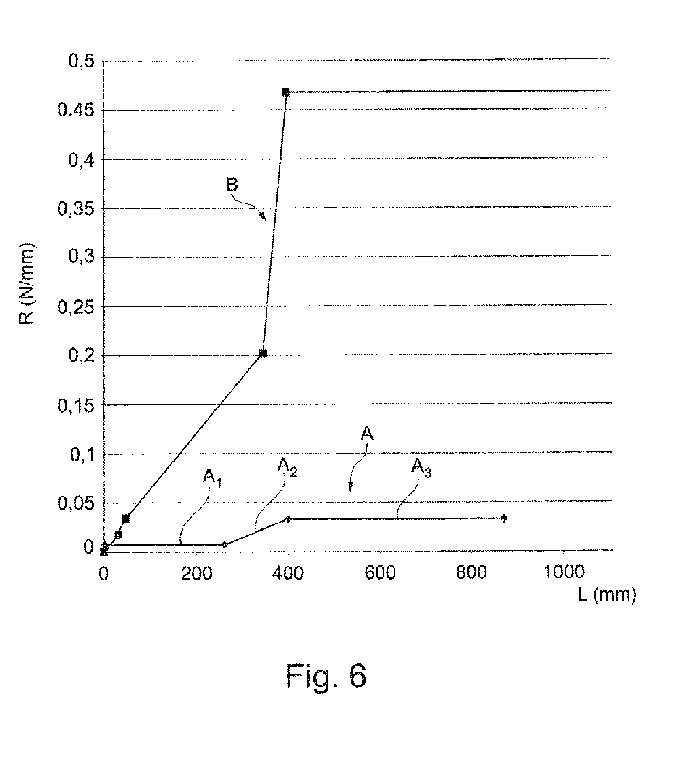

A cet égard, on entendra ici par "raideur" (ou, inversement, "souplesse") essentiellement la raideur en flexion : la souplesse en flexion de la partie distale 22 permet d'aller dans le réseau profond, tandis que la raideur en flexion de la partie proximale 26 procure de la "pushabilité", c'est-à-dire l'aptitude à faire progresser le microcathéter dans le sens de sa longueur sans arc-boutement. En ce qui concerne la "torquabilité", c'est-à-dire l'aptitude à transmettre jusqu'à l'extrémité distale un couple de rotation appliqué à l'extrémité proximale, la raideur de la partie proximale y contribue certes, mais cette fonction est principalement assurée par un fil-guide introduit dans la lumière centrale du microcathéter pendant l'implantation. La Figure 6 illustre la caractéristique de variation de la raideur, mesurée en N/mm en fonction de l'abscisse par rapport à l'extrémité distale (longueur L, mesurée en mm).

Il s'agit ici de la raideur en flexion, mesurée par un test de raideur trois points selon ASTM D 790 ou ISO 178. Dans ce test, l'échantillon est posé entre deux points, et une machine de traction dynamométrique impose à l'échantillon une déformation sous l'effet d'une force appliquée en un troisième point, central, jusqu'à atteindre une flèche de 5 mm.

La caractéristique A, relevée pour un microcathéter selon l'invention, comprend :
- une partie A1 correspondant à la région cylindrique distale 22 de diamètre 0,8 mm : raideur faible et constante de 0,007 N/mm ± 10 % ;
- une partie A2 correspondant à la région conique de transition 24 : gradient de raideur évolutif et linéaire compris entre 0,007 et 0,034 N/mm, avec une valeur centrale de 0,017 N/mm ; et
- une partie A3 correspondant à la région cylindrique proximale 26 de diamètre 1,3 mm : raideur forte et constante de 0,034 N/mm ± 10 %.

Par comparaison, on a porté sur ce même graphique la caractéristique B correspondant à un mandrin, en l'espèce un mandrin (fil-guide) de type *BMW Universal* de Abbott Vascular, de diamètre 0,35 mm. En effet, dans la mesure où il n'existe pas, actuellement, de cathéter aussi fin que le microcathéter de l'invention (les cathéters conventionnels ne permettent pas d'atteindre le réseau coronaire profond, à plus forte raison ne permettent pas d'atteindre et de traverser les anastomoses), la seule comparaison possible avec un accessoire de diamètre comparable est une comparaison avec un mandrin, dont on notera toutefois qu'il ne s'agit pas d'un accessoire implantable.

Un tel mandrin présente une raideur variable, résultant d'une succession de matériaux de raideurs et de formes différentes (cylindres pleins juxtaposés, puis ressort pour la partie distale, la plus souple). Les raideurs relevées pour un tel mandrin sont des raideurs variables (sauf pour la zone distale), s'étageant de 0,001 à 0,46 N/mm, dans des plages de valeurs beaucoup plus élevées que celles du microcathéter de l'invention.

On a illustré Figure 7, isolément, une microsonde utilisable avec le microcathéter de l'invention, et Figure 8 cette même microsonde insérée dans le microcathéter.

La microsonde 10 est constituée d'un microcâble 36 dont l'extrémité distale 38, qui constitue la partie électriquement active, est pourvue d'une ou plusieurs préformes bi- ou tridimensionnelles, par exemple deux ondulations 40 dans l'exemple illustré. Le microcâble 36 est pourvu d'un revêtement isolant sur toute sa longueur, à l'exception de zones ponctuellement dénudées destinées à constituer les électrodes de détection/stimulation, par exemple les zones ponctuellement dénudées 42 situées aux sommets de chacune des ondulations 40. L'extrémité proximale, quant à elle, est pourvue d'un connecteur de liaison 44, par exemple un connecteur de type IS-1 ou IS-4 pour sonde monopolaire.

La Figure 8 illustre la microsonde 10 insérée dans le microcathéter 12 de la Figure 3. Dans l'exemple illustré, la partie distale active 38 de la microsonde a été déployée de manière à émerger du débouché 34 du microcathéter 12. On dispose ainsi d'un ensemble télescopique microcathéter/microsonde, avec possibilité de déplacement axial relatif entre le microcathéter 12 et la microsonde 10. Cette action permet au chirurgien de choisir la longueur émergente découvrant le nombre d'électrodes le plus approprié au traitement envisagé. Le mouvement est bien entendu réversible, à la discrétion du chirurgien.

Plus précisément, pour son implantation, la microsonde est implantée par une technique conventionnelle de type OTW ou "filoguidage" au moyen d'un mandrin très fin formant fil-guide, pourvu à son extrémité distale d'une terminaison atraumatique permettant son introduction directe dans les vaisseaux du réseau coronaire sans risque de perforation.

Au préalable, le chirurgien dispose un cathéter principal lui permettant d'accéder au déboucher du sinus coronaire CS. Un cathéter de sous-sélection est ensuite utilisé pour choisir, sous amplificateur de brillance, le chemin du réseau coronaire veineux qui permettra d'atteindre la veine cible.

Le chirurgien introduit alors le mandrin dans le cathéter de sous-sélection, et pousse ce mandrin pour le faire progresser, à nu, dans le réseau veineux coronaire de façon à sélectionner telle ou telle veine collatérale, éventuellement jusqu'à une anastomose avec traversée de cette anastomose et remontée dans la veine "retour" choisie.

Le chirurgien enfile ensuite sur le mandrin le microcathéter 12 selon l'invention, qu'il fait glisser et progresser sur ce mandrin jusqu'à l'extrémité de ce dernier.

Après retrait du mandrin, le chirurgien enfile la microsonde 10 dans le microcathéter 12 à partir de l'extrémité proximale de ce dernier, puis pousse la microsonde 10 sur toute la longueur du microcathéter. Ce dernier est ensuite reculé pour découvrir à volonté les portions ondulées 40 de la partie active 38 de la microsonde, et donc les électrodes 42 de la partie active stimulante de la microsonde, qui vont se trouver disposées aux sites de stimulation respectifs choisis.

Comme on peut le constater, ces étapes de canulation de veine sont des manipulations couramment pratiquées par les spécialistes, de sorte que l'implantation d'une microsonde et d'un microcathéter selon l'invention ne nécessite l'apprentissage d'aucune nouvelle technique opératoire ni habileté particulière.

Comme on l'a indiqué en introduction, en raison de la présence du connecteur 44 relié à la microsonde 10, il n'est pas possible de retirer le microcathéter qui, compte tenu de son faible diamètre, ne peut pas être réalisé selon les techniques conventionnelles (cathéter pelable, etc.).

Le microcathéter 12 doit donc être laissé en place, implanté avec la microsonde 10. Pour éviter tout déplacement entre ces deux éléments, il est nécessaire de procéder à une immobilisation finale du microcathéter sur la microsonde.

Cette immobilisation peut être réalisée par une simple ligature, correspondant à l'exemple de réalisation des Figures 9a et 9b qui sont des vues de détail, respectivement en perspective et en coupe, montrant l'extrémité proximale du microcathéter, où celui-ci est assujetti à la microsonde par ligature.

L'élément 18 emboité à l'extrémité du tube 14 du microcathéter est pourvu à sa surface extérieure d'une pluralité de gorges 46 et comporte à son extrémité la plus distale un orifice 48 de faible diamètre permettant le passage et le glissement du corps de la microsonde 10. Le verrouillage axial de la microsonde au microcathéter est obtenu par un effort de serrage au moyen d'un ou plusieurs fils de ligature (non représentés) d'un type utilisé couramment pour ligaturer une sonde à une veine. Ce verrouillage est réalisé par le chirurgien après la manoeuvre télescopique de la microsonde dans le microcathéter, après s'être assuré du bon positionnement des électrodes et de l'efficacité de la stimulation. L'élément 18 est réalisé dans une matière suffisamment souple, par exemple en silicone, pour permettre sa déformation sous l'action du serrage du fil de ligature. Les trois gorges 46 ont pour fonction de permettre la mise en place des fils de ligature et de répartir la zone de serrage de la microsonde sur un maximum de surface, la réduction d'épaisseur en fond de gorge facilitant la transmission de l'effort de serrage.

Les Figures 10a et 10b illustrent une première variante de l'élément 18, comportant deux pièces mâle 50 et femelle 52 ayant la possibilité de se déplacer axialement par vissage. L'extrémité 54 de la pièce mâle prend appui sur une région intérieure conique 56 de la pièce femelle, ce qui entraine localement une réduction du diamètre et le pincement de la microsonde 10. Le blocage final du microcathéter sur la microsonde est réalisé par vissage.

Les Figures 11a et 11b illustrent une deuxième variante, qui diffère de la précédente par le mode d'assemblage de la partie mâle 50 et de la partie femelle 52, ici réalisé par des clips 58 venant se loger dans des orifices homologues 60. Le serrage du microcathéter sur la microsonde est réalisé par translation axiale de la pièce mâle 50 dans la pièce femelle 52, le blocage étant signalé par l'encliquetage des clips 58. Ici encore, la pièce mâle est pourvue de surfaces coniques 62 coopérant avec des surfaces homologues 64 de la pièce femelle de manière à réaliser une réduction du diamètre interne et un pincement de la microsonde 10.

Divers perfectionnements peuvent être apportés au microcathéter de l'invention, pour tirer notamment parti de la conception en deux éléments distincts (microsonde/microcathéter), ce qui augmente la modularité : en particulier, une famille de microsondes de longueurs différentes peut être utilisée avec un même modèle de microcathéter implantable.

Le microcathéter peut être également pourvu, en partie distale, d'un connecteur tel que celui illustré en 66 sur les Figures 12 et 13. Ce connecteur permet d'injecter un produit (héparine notamment, ou produit de contraste) lors de la mise en place du microcathéter, avant enfilage de la microsonde. L'extrémité distale 68 est conformée à cet effet avec une géométrie permettant le remplissage par un liquide après emboitement d'un accessoire 70 garantissant l'étanchéité, notamment en prévoyant dans la lumière interne un relief circulaire 72 formant joint d'étanchéité. Un cône d'entrée 74 permet de guider l'accessoire 70 lors de son insertion.

## Revendications

1. Un cathéter (12), comprenant un tube creux (14) avec une lumière centrale (28) s'étendant sur toute la longueur du tube depuis une région proximale (26) jusqu'à une région distale (22), dans lequel :
- le diamètre externe du tube du cathéter dans la région distale (22) est au plus égal à 1 mm (3 French) ;
- le diamètre de la lumière centrale du tube du cathéter est au moins égal à 0,2 mm ;
- la raideur en flexion de la région proximale (26) est supérieure à la raideur en flexion de la région distale (22), et le tube comporte une région de transition (24) présentant un gradient de raideur décroissant depuis la région proximale (26) vers la région distale (22) ; et
- le cathéter porte dans la région distale des éléments radio-opaques (16) ou des références visuelles,
**caractérisé en ce que** :
- la paroi du tube est dépourvue d'armure de rigidification ;
- la région distale (22) présente une raideur constante ; et
- le cathéter est réalisé en matériau(x) biocompatible(s) adapté(s) à une implantation permanente dans des réseaux veineux, artériels ou lymphatiques.

2. Le cathéter de la revendication 1, dans lequel le tube présente un gradient d'épaisseur décroissante, de la région proximale vers la région distale du cathéter.

3. Le cathéter de la revendication 2, dans lequel le tube comporte une portion (24) de diamètre extérieur (0) décroissant de la région proximale vers la région distale du cathéter, le diamètre intérieur de la lumière centrale étant constant sur toute la longueur du tube.

4. Le cathéter de la revendication 3, dans lequel le tube comprend un noyau tubulaire (30) de diamètre extérieur constant revêtu d'un matériau de gainage (32) d'épaisseur variable, cette épaisseur augmentant progressivement de la région distale vers la région proximale du cathéter.

5. Le cathéter de la revendication 3, dans lequel ladite portion (24) de diamètre extérieur décroissant est une portion conique, de diamètre (0) uniformément décroissant.

6. Le cathéter de la revendication 3, dans lequel ladite portion de diamètre extérieur décroissant est une portion médiane (24) du cathéter, prolongée côté proximal par une portion (26) de diamètre constant et côté distal par une portion (22) de diamètre constant, l'épaisseur et le diamètre extérieur du tube dans la région proximale étant supérieurs à l'épaisseur et au diamètre extérieur du tube dans la région distale

7. Le cathéter de la revendication 1, dans lequel la raideur en flexion de la partie distale (22) n'est pas supérieure à 0,09 N/mm.

8. Le cathéter de la revendication 1, dans lequel le ratio entre la raideur en flexion de la partie proximale (26) et la raideur en flexion de la partie distale (22) est compris entre 2 et 10.

9. Le cathéter de la revendication 1, dans lequel le tube présente dans la direction de sa longueur une structure sans discontinuité et sans soudure.

10. Le cathéter de la revendication 1, dans lequel le tube comprend dans la direction de sa longueur une structure formée d'une série de tubes de diamètres respectifs différents, réunis les uns à la suite des autres.

11. Le cathéter de la revendication 1, comprenant en outre dans la région proximale un organe (18) de solidarisation à un corps d'une sonde apte à être introduite dans la lumière du cathéter.

12. Le cathéter de la revendication 9, dans lequel l'organe de solidarisation (18) est un embout radialement compressible, apte à recevoir une ligature.

13. Le cathéter de la revendication 9, dans lequel l'organe de solidarisation est une pince comportant une pièce centrale (50) de pincement pourvue de mors déformables (54, 62), entourée par une pièce (52) de serrage desdits mors.

14. Le cathéter de la revendication 1, dans lequel la surface intérieure de la lumière centrale est une surface d'un matériau antifriction différent du matériau formant le reste du tube.

15. Le cathéter de la revendication 1, portant en outre dans une région centrale du tube un manchon de retenue (20) apte à assujettir le cathéter à la paroi d'un vaisseau dans une région distante de la région distale.
